# EUROPEAN PATENT APPLICATION

(11) **EP 4 797 069 A1**
(43) Date of publication of application: **26.08.2026**
(21) Application number: 26158018.7
(22) Date of filing: 12.02.2026
(51) Int. Cl.: G06F 3/16

(54) **ANALYSIS SUPPORT SYSTEM, ANALYSIS SUPPORT METHOD, AND PROGRAM**

(30) Priority: 12.02.2025 JP 2025020759
(71) Applicant: SHIMADZU CORPORATION, Kyoto-shi, Kyoto 604-8511 (JP)
(72) Inventor: YOSHIZAKO, Mitsutomo, Kyoto (JP)
(74) Representative: Müller-Boré & Partner Patentanwälte PartG mbB

(57) **Abstract**

An analysis support system (1) is an analysis support system (1) for supporting an analysis task in an analysis apparatus (200), comprising: a voice device (10) configured to be worn by each analyst using the analysis apparatus (200) and to perform analysis support by voice; and a terminal device (100) provided corresponding to the analysis apparatus (200) and connected to the voice device (10) via wireless communication. The terminal device (100) includes: a storage unit configured to store support information for the analysis apparatus (200) and analyst information including a proficiency of the analyst in the analysis task; a communication unit configured to authenticate wireless communication with the voice device (10) of the analyst who is within a predetermined range of the analysis apparatus (200); and a terminal processing unit configured to identify the analyst authenticated by the communication unit and capable of wireless communication, and to output the support information for the analysis apparatus (200) corresponding to the proficiency of the identified analyst to the voice device (10).

## Description

### [Technical Field]

The present disclosure relates to an analysis support system, an analysis support method, and a program.

### [Background Art]

An example of a liquid chromatograph, which is an analysis apparatus, is disclosed in JP 2024-9363 A (Patent Literature 1). The liquid chromatograph disclosed in Patent Literature 1 is used to identify or quantify components in a sample.

### [Citation List]

### [Patent Literature]

Patent Literature 1: JP 2024-9363 A

### [Summary of Invention]

### [Technical Problem]

In order to perform analysis with an analysis apparatus such as the liquid chromatograph disclosed in Patent Literature 1, it is necessary to perform various analysis tasks. Examples include preparation of samples, confirmation of analysis results, and replacement of consumables.

It is desirable to provide analysis support so that even an analyst with little experience in analysis tasks can perform such analysis tasks accurately and efficiently. Further, even for an analyst with abundant experience in analysis tasks, analysis support is desired depending on the content and situation of the analysis tasks.

The present disclosure has been made to solve such problems, and an object thereof is to make it possible to provide necessary analysis support to an analyst.

### [Solution to Problem]

An analysis support system according to the present disclosure is an analysis support system for supporting an analysis task in an analysis apparatus, comprising: a voice device configured to be worn by each analyst using the analysis apparatus and to perform analysis support by voice; and a terminal device provided corresponding to the analysis apparatus and connected to the voice device via wireless communication. The terminal device includes: a storage unit configured to store support information for the analysis apparatus and analyst information including a proficiency of the analyst in the analysis task; a communication unit configured to authenticate wireless communication with the voice device of the analyst who is within a predetermined range of the analysis apparatus; and a terminal processing unit configured to identify the analyst authenticated by the communication unit and capable of wireless communication, and to output the support information for the analysis apparatus corresponding to the proficiency of the identified analyst to the voice device.

### [Advantageous Effects of Invention]

According to the present disclosure, it is possible to provide necessary analysis support to an analyst.

### [Brief Description of Drawings]

Fig. 1 is a diagram schematically showing a configuration of an analysis support system according to Embodiment 1.
Fig. 2 is a diagram schematically showing a wearing state and a configuration of a voice device.
Fig. 3 is a block diagram of the voice device and a terminal device.
Fig. 4 is a diagram showing an example of analyst information.
Fig. 5 is a flowchart showing an example of analysis support processing executed by the analysis support system according to Embodiment 1.
Fig. 6 is a diagram showing an example of an image for accepting an input of a work achievement level of an analyst.
Fig. 7 is a diagram showing an example of an image corresponding to the lowest proficiency among support information.
Fig. 8 is a diagram schematically showing a configuration of an analysis support system according to Embodiment 2.
Fig. 9 is a diagram showing a state where an analyst has moved in the analysis support system according to Embodiment 2.
Fig. 10 is a block diagram of a management device.
Fig. 11 is a diagram showing an example of an image displaying the proficiency of an analyst and an analysis status of an analysis apparatus.
Fig. 12 is a diagram showing an example of an image for accepting an input of a physical condition of an analyst.
Fig. 13 is a diagram showing an example of an image indicating measured values of a biological state and a physical condition of an analyst.
Fig. 14 is a diagram showing an example of processing of an analysis support system according to Modification 2 of Embodiment 2.
Fig. 15 is a diagram showing an example of support information possessed by a terminal device according to Modification 2 of Embodiment 2.

### [Description of Embodiments]

In order to perform analysis with an analysis apparatus such as a liquid chromatograph, it is necessary to perform various analysis tasks. Examples include preparation of samples, confirmation of analysis results, and replacement of consumables. These analysis tasks include many procedures. In addition, there are matters to be noted in each procedure so that the analysis apparatus can execute analysis normally. Furthermore, various analysis apparatuses are generally placed in a laboratory where an analyst performs analysis tasks, and the analysis tasks differ for each analysis apparatus.

An analyst with little experience in analysis tasks may work while checking a work manual on a PC monitor or the like in order to perform the analysis tasks accurately. Even an analyst with abundant experience in analysis tasks may similarly work while checking a work manual if they have forgotten the procedures because they have not performed the target analysis task for a long period of time. In these cases, the work stops every time the analyst operates the PC or views the work manual, so the analyst cannot work efficiently. Therefore, it is desirable to provide necessary analysis support to the analyst so that the analyst can perform the analysis tasks accurately and efficiently.

### [Embodiment 1]

### [Configuration of Analysis Support System]

Hereinafter, the present embodiment will be described in detail with reference to the drawings. Note that in the following, the same or corresponding parts in the drawings are denoted by the same reference numerals, and description thereof will not be repeated.

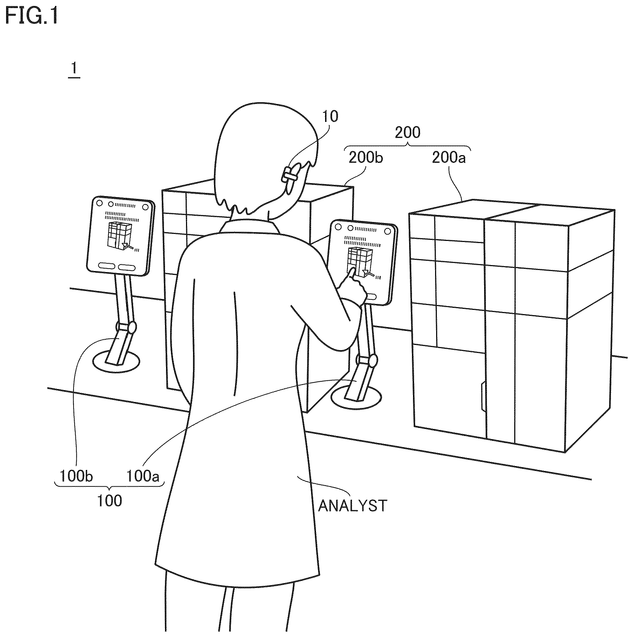

Fig. 1 is a diagram schematically showing a configuration of an analysis support system 1 according to Embodiment 1. The analysis support system 1 includes a voice device 10 and a terminal device 100.

The analysis support system 1 supports an analyst in performing an analysis task on an analysis apparatus 200. The analysis apparatus 200 is, for example, a liquid chromatograph.

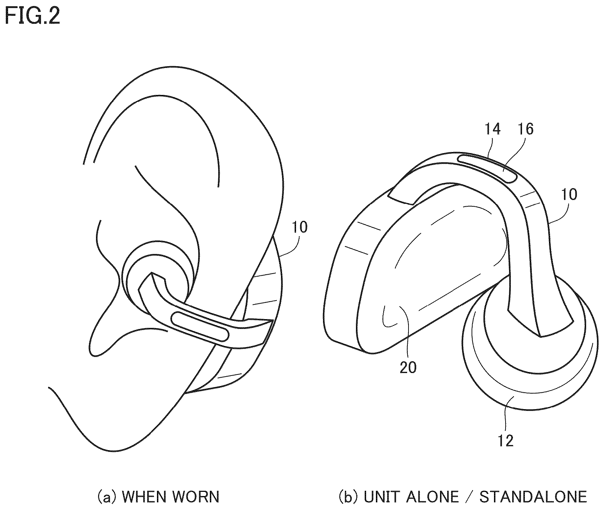

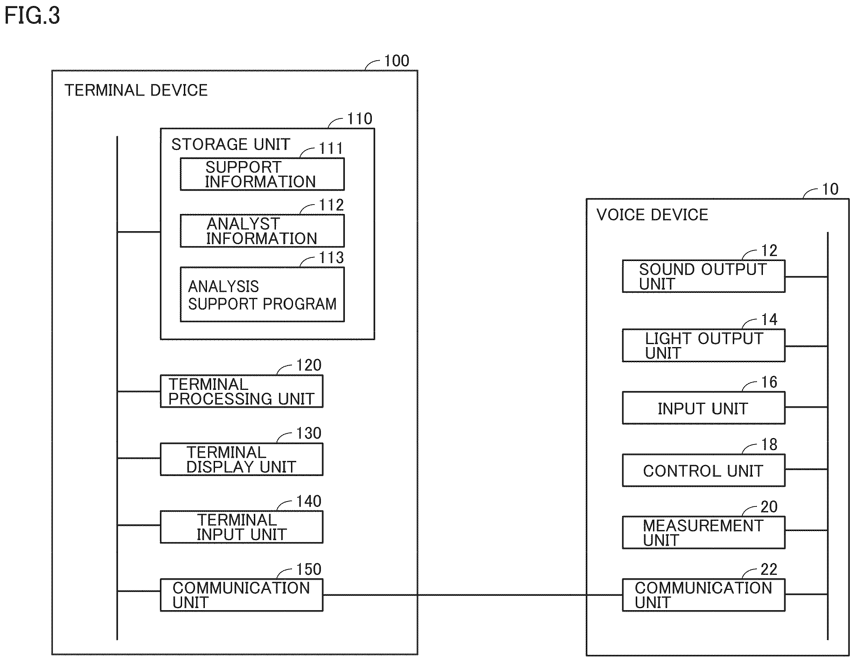

The voice device 10 is worn by the analyst. Fig. 2 is a diagram schematically showing a wearing state and a configuration of the voice device 10. As shown in Fig. 2(a), the voice device 10 of the present embodiment is an open-type earphone. Fig. 3 is a block diagram of the voice device 10 and the terminal device 100. The voice device 10 includes a sound output unit 12, a light output unit 14, an input unit 16, a control unit 18, a measurement unit 20, and a communication unit 22.

The sound output unit 12 outputs voice to the analyst wearing the voice device 10. The sound output unit 12 is composed of, for example, a diaphragm that generates sound.

The light output unit 14 outputs light indicating an operating state of the voice device 10. The operating state of the voice device 10 is, for example, a state where the power is on, or a state where voice is being output. The light output unit 14 is composed of, for example, an LED.

The input unit 16 accepts an input from the analyst to the voice device 10. The input to the voice device 10 is, for example, an input for causing the output voice to be output again. The input unit 16 is composed of, for example, a touch sensor. Furthermore, the input to the voice device 10 may be a voice input. In that case, the input unit 16 is composed of a microphone.

The measurement unit 20 acquires measured values such as body temperature or heart rate, which are biological states of the analyst. The measurement unit 20 is composed of, for example, a thermometer or a heart rate monitor.

The communication unit 22 and a communication unit 150 of the terminal device 100 described later connect the voice device 10 and the terminal device 100 via wireless communication. In the present embodiment, the communication unit 22 is composed of a Bluetooth (registered trademark) antenna.

The control unit 18 is composed of a processor and a memory. The control unit 18 operates the sound output unit 12, the light output unit 14, the input unit 16, the control unit 18, the measurement unit 20, and the communication unit 22.

The terminal device 100 includes a storage unit 110, a terminal processing unit 120, a terminal display unit 130, a terminal input unit 140, and a communication unit 150.

The terminal device 100 is provided corresponding to the analysis apparatus 200. In the present embodiment, as shown in Fig. 1, a terminal device 100a is provided corresponding to an analysis apparatus 200a, and a terminal device 100b is provided corresponding to an analysis apparatus 200b.

The storage unit 110 is composed of a volatile memory such as a DRAM (Dynamic Random Access Memory) or an SRAM (Static Random Access Memory), or a nonvolatile storage device such as a ROM (Read Only Memory), a flash memory, an HDD (Hard Disk Drive), or an SSD (Solid State Drive). The storage unit 110 stores support information 111, analyst information 112, and an analysis support program 113.

The analyst information 112 includes identification information of the voice device 10, identification information of the analyst corresponding to the identification information of the voice device 10, and, regarding the analysis task on the analysis apparatus 200 to which the terminal device 100 corresponds, the proficiency of the analyst, the previous execution date, the past number of executions, and the previous work achievement level.

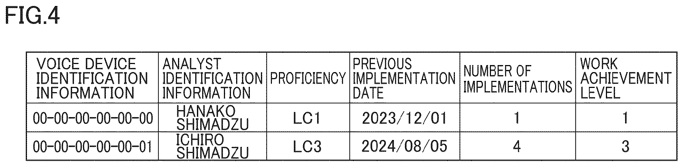

Fig. 4 is a diagram showing an example of the analyst information 112. In Fig. 4, the identification information of the voice device 10 is a MAC address used in Bluetooth communication. The identification information of the analyst is the name of the analyst, but may be, for example, a unique number.

In the present embodiment, the proficiency of the analyst in the analysis task of the analysis apparatus 200 has five levels from LC1 to LC5. The proficiency LC1 is the lowest, and the higher the number, the higher the proficiency. As will be described later, the proficiency is updated based on the previous execution date, the past number of executions, and the previous work achievement level regarding the analysis task on the analysis apparatus 200 to which the terminal device 100 corresponds.

The support information 111 includes voice information indicating procedures and precautions for the analysis task on the analysis apparatus 200 to which the terminal device 100 corresponds. The procedures for the analysis task include each task from the start to the end of the analysis task and the order thereof. The support information 111 differs for each proficiency of the analyst in the analysis task.

Examples of the voice information corresponding to the lowest proficiency among the support information 111 include Voice Information 01 "Please take out the piping from the column oven", Voice Information 02 "Please connect the piping and the column from the IN side", and Voice Information 03 "When starting liquid flow, please set the flow rate to a pressure less than half that of normal analysis. Sudden pressure changes may lead to column degradation." Of these, Voice Information 01 and Voice Information 02 indicate the procedures of the analysis task. Voice Information 03 indicates precautions in the analysis task.

For voice information corresponding to higher proficiency, for example, Voice Information 03, which is a precaution, is omitted from the above example of voice information, so that there are fewer precautions compared to when the proficiency is lower. Thus, the support information for each proficiency is support information necessary and sufficient for an analyst having that proficiency to perform the analysis task accurately.

The analysis support program 113 is a program for operating the terminal processing unit 120 when providing analysis support to the analyst.

The terminal processing unit 120 is a computer that reads the analysis support program 113 stored in the storage unit 110, expands the read program in the storage unit 110, and executes it. The terminal processing unit 120 is composed of, for example, a CPU (Central Processing Unit), an FPGA (Field Programmable Gate Array), a GPU (Graphics Processing Unit), or an MPU (Multi Processing Unit). Note that the terminal processing unit 120 may be composed of processing circuitry.

The communication unit 150 is composed of a Bluetooth antenna so as to be able to communicate with the communication unit 22 of the voice device 10. The communication unit 150 of the terminal device 100 connects via wireless communication with the voice device 10 worn by an analyst who is within a predetermined range of the analysis apparatus 200 to which the terminal device 100 corresponds. At the same time, the communication unit 150 of the terminal device 100 does not connect via wireless communication with a voice device 10 worn by an analyst who is within a predetermined range of an analysis apparatus 200 to which the terminal device 100 does not correspond.

For example, the predetermined range of the analysis apparatus 200a is the range where the analyst is present when the analyst performs an analysis task on the analysis apparatus 200a. That is, the range in which the communication unit 150 of the terminal device 100a connects via wireless communication is set to include the range where the voice device 10 worn by the analyst exists when the analyst performs an analysis task on the analysis apparatus 200a. At the same time, the range in which the communication unit 150 of the terminal device 100a connects via wireless communication is set not to include the range where the voice device 10 worn by the analyst exists when the analyst performs an analysis task on the analysis apparatus 200b.

The communication unit 150 is pre-paired with the communication unit 22 of the analyst's voice device 10 in Bluetooth communication. That is, the communication unit 150 of the terminal device 100 acquires the MAC address of the voice device 10 of the analyst who is within the predetermined range of the analysis apparatus 200 to which the terminal device 100 corresponds, authenticates whether or not the voice device 10 has been paired in advance based on the acquired MAC address, and connects to the voice device 10 via wireless communication if authentication is successful.

Furthermore, the communication unit 150 is set to connect with the voice device 10 on a one-to-one basis. For example, when the communication unit 150 is connected to the voice device 10 of an analyst within the predetermined range of the analysis apparatus 200, even if another analyst enters the predetermined range of the analysis apparatus 200, the communication unit 150 does not connect to the voice device 10 of the other analyst.

In the present embodiment, the terminal display unit 130 and the terminal input unit 140 are integrally configured as a touch panel. The terminal display unit 130 displays an image on the panel of the touch panel. The terminal input unit 140 accepts an input from the analyst by detecting a touch on the touch panel.

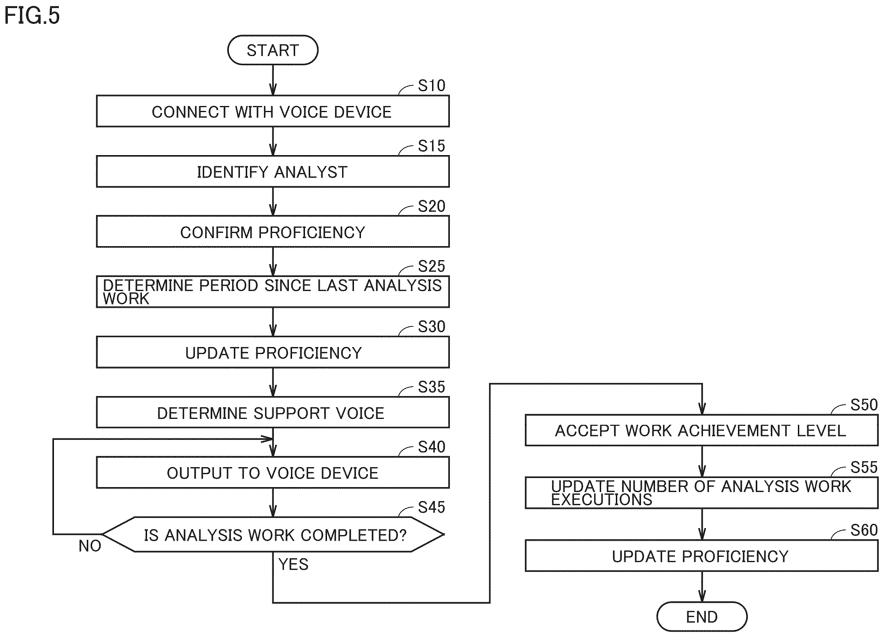

[Analysis Support Method] Processing related to the analysis support method executed by the analysis support system 1 will be described with reference to the drawings. Fig. 5 is a flowchart showing an example of analysis support processing executed by the analysis support system 1 according to Embodiment 1. Note that each step shown in Fig. 5 is realized by the terminal processing unit 120 executing the analysis support program 113. This flowchart is started when an analyst wearing the voice device 10 enters the predetermined range of the analysis apparatus 200, that is, when the communication unit 150 acquires the MAC address of the voice device 10. It is assumed that pairing in Bluetooth communication between the communication unit 150 of the terminal device 100 and the communication unit 22 of the voice device 10 has already been performed.

First, the terminal processing unit 120 causes the communication unit 150 to establish a connection with the voice device 10 via wireless communication (Step S10). Specifically, the terminal processing unit 120 causes the communication unit 150 to authenticate whether or not the voice device 10 has been paired in advance based on the acquired MAC address of the voice device 10, and if authentication is successful, causes it to connect to the voice device 10 via wireless communication.

Next, the terminal processing unit 120 identifies the analyst wearing the voice device 10 that has been authenticated and established wireless communication (Step S15). Specifically, the terminal processing unit 120 reads the analyst information 112 stored in the storage unit 110, and identifies the identification information of the analyst corresponding to the identification information of the voice device 10 with which wireless communication was established, based on the read analyst information 112.

Next, the terminal processing unit 120 checks the identified analyst's proficiency in the analysis task (Step S20). Specifically, the terminal processing unit 120 checks the proficiency of the analyst corresponding to the identified identification information of the analyst in the analysis task, based on the read analyst information 112.

Next, the terminal processing unit 120 determines the period from the previous analysis task of the identified analyst (Step S25). Specifically, the terminal processing unit 120 checks the execution date of the previous analysis task of the identified analyst based on the read analyst information 112, and determines the period from the execution date of the previous analysis task to the execution date of the current analysis task.

Next, the terminal processing unit 120 updates the identified analyst's proficiency in the analysis task (Step S30). Specifically, the terminal processing unit 120 updates the proficiency such that the proficiency decreases as the period from the previous analysis task becomes longer, and updates the proficiency such that the proficiency increases as the period from the previous analysis task becomes shorter. For example, if the period from the previous analysis task is 6 months, the proficiency is lowered by one level, and if the period from the previous analysis task is 2 weeks, the proficiency is raised by one level.

Next, the terminal processing unit 120 determines support information for the analysis task of the identified analyst (Step S35). Specifically, the terminal processing unit 120 reads the support information 111 stored in the storage unit 110, and determines support information corresponding to the updated proficiency of the identified analyst based on the read support information 111.

Next, the terminal processing unit 120 causes the determined support information to be output to the voice device 10 (Step S40). Specifically, the terminal processing unit 120 transmits the determined support information to the control unit 18 of the voice device 10 via the communication unit 150 of the terminal device 100 and the communication unit 22 of the voice device 10. The control unit 18 of the voice device 10 causes the sound output unit 12 to output the transmitted support information. At this time, the terminal processing unit 120 causes the voice device 10 to output support information regarding the first procedure of the analysis task.

Next, the terminal processing unit 120 determines whether or not the analysis task is completed (Step S45). Specifically, the terminal processing unit 120 checks whether or not the determined support information has been output up to the last procedure. If it is not confirmed that the determined support information has been output up to the last procedure, the terminal processing unit 120 determines that the analysis task is not completed (NO in Step S45). Then, the terminal processing unit 120 causes support information regarding the procedure subsequent to the already output support information to be output (Step S40). If it is confirmed that the determined support information has been output up to the last procedure, the terminal processing unit 120 determines that the analysis task is completed (YES in Step S45).

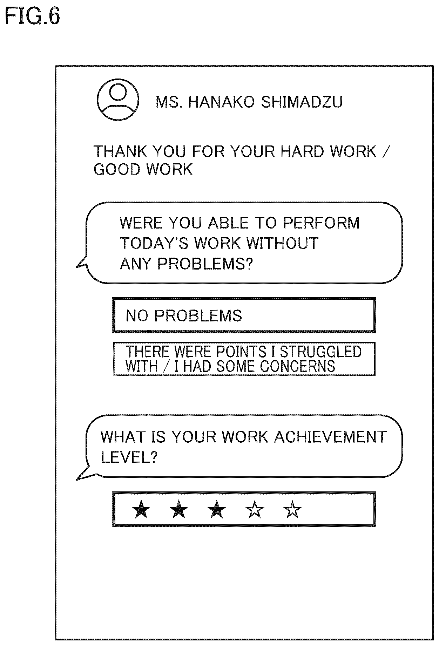

Next, when it is determined that the analysis task is completed, the terminal processing unit 120 accepts an input of the analyst's work achievement level (Step S50). Specifically, the terminal processing unit 120 causes the terminal display unit 130 to display an image for accepting an input of the analyst's work achievement level. Then, the terminal processing unit 120 causes the terminal input unit 140 to accept the input of the analyst's work achievement level. Fig. 6 is a diagram showing an example of an image for accepting an input of the presence or absence of a problem in the day's work and the work achievement level of the analyst. In this example, the analyst selects "There were no problems" or "There were points to worry about" regarding the day's work, and evaluates and inputs the work achievement level in one of 5 levels.

Next, the terminal processing unit 120 updates the number of times the analysis task has been performed by the analyst (Step S55). Specifically, the terminal processing unit 120 updates the number of executions of the analysis task performed by the analyst in the analyst information 112 so as to increase it by one.

Finally, the terminal processing unit 120 updates the proficiency (Step S60). Specifically, the terminal processing unit 120 updates the proficiency based on the accepted work achievement level and the updated number of executions of the analysis task. At this time, the terminal processing unit 120 updates the proficiency such that the higher the work achievement level, the higher the proficiency, and the greater the number of executions, the higher the proficiency. For example, if the work achievement level continues to be level 3 three times, the proficiency is raised by one level, and if the work achievement level is level 5, the proficiency is raised by one level in one time. Conversely, if the work achievement level continues to be level 2 three times, the proficiency is lowered by one level, and if the work achievement level is level 1, the proficiency is lowered by one level in one time.

As described above, the analysis support system 1 according to the present embodiment outputs support information of the analysis apparatus corresponding to the proficiency of the analyst performing the analysis task to the voice device 10 worn by the analyst. Since the support information corresponds to the proficiency of the analyst, the analyst can obtain support information necessary and sufficient for working accurately. In addition, since the support information is output to the voice device 10 worn by the analyst, the analyst can obtain the support information while working. As a result, the analysis support system 1 can provide necessary analysis support to the analyst.

Further, the analysis support system 1 according to the present embodiment sets the proficiency of the analyst in the analysis task as a proficiency based on the past number of executions of the analysis task, the period from the previous analysis task, and the work achievement level of the previous analysis task. Thereby, the analyst can obtain support information corresponding to a proficiency based on the analyst's past number of executions of the analysis task, the period from the previous analysis task, and the work achievement level of the previous analysis task.

Furthermore, in the analysis support system 1 according to the present embodiment, since the voice device 10 is an open-type earphone, the analyst can perform the analysis task while listening to ambient sounds. For example, the analyst can perform the analysis task while checking the analysis status of the analysis apparatus 200 by the sound emitted by the analysis apparatus 200.

### <Modification 1 of Embodiment 1>

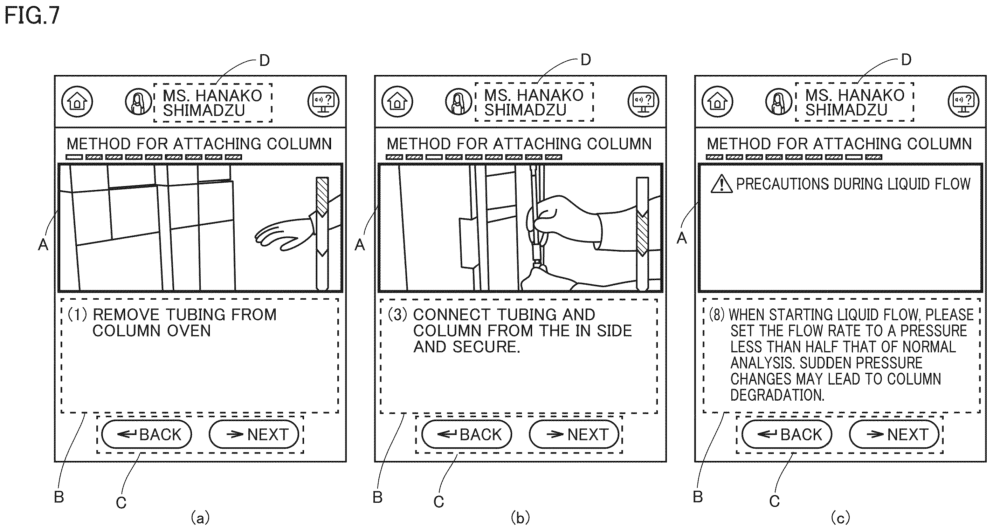

In the above-described embodiment, the support information 111 is voice information. However, the support information 111 may further include image information. Fig. 7 is a diagram showing an example of an image corresponding to the lowest proficiency among the support information 111. Fig. 7(a) and Fig. 7(b) show the procedures of the analysis task. Fig. 7(c) shows precautions in the analysis task. Part A of Fig. 7 is an image showing a state of working on the analysis apparatus 200. Part B of Fig. 7 is a character image showing the procedure or precaution of the analysis task.

For an image corresponding to higher proficiency, for example, Fig. 7(c) showing precautions in Fig. 7 is omitted, so that there are fewer precautions compared to when the proficiency is lower. Thus, the image information for each proficiency is support information necessary and sufficient for an analyst having that proficiency to perform the analysis task accurately, similarly to the voice information.

In this case, in the processing of determining the support information (Step S35), the terminal processing unit 120 further determines image information as the support information. Then, in the processing of causing the determined support information to be output to the voice device 10 (Step S40), the terminal processing unit 120 causes the image information to be output to the terminal display unit 130 in accordance with causing the voice information to be output to the voice device 10.

Thereby, the analyst can check the image of the support information on the terminal display unit 130 while listening to the voice of the support information on the voice device 10. As a result, for example, even if the voice is missed, the analysis task can be performed by checking the image.

### <Modification 2 of Embodiment 1>

In Modification 1 described above, the terminal device 100 may further accept an input from the analyst and switch the output support information based on the accepted input. Specifically, the terminal display unit 130 outputs an image indicating a touch location for accepting an input. Then, the terminal input unit 140 detects that a touch has been made. The image of part C in Fig. 7 is an image showing "Back" and "Next" which are touch locations. When the terminal input unit 140 detects that "Next" in Fig. 7 is touched, the terminal processing unit 120 switches the support information being output to support information regarding the procedure subsequent to the current procedure. Conversely, when the terminal input unit 140 detects that "Back" in Fig. 7 is touched, the terminal processing unit 120 switches the support information being output to support information regarding the procedure preceding the current procedure.

Thereby, the analyst can switch the output support information in accordance with the progress of the actual analysis task.

### <Modification 3 of Embodiment 1>

In Modification 1 described above, the terminal device 100 may further cause the terminal display unit 130 to output an image indicating the identification information of the identified analyst. The character image "Ms. Hanako Shimadzu" in part D of Fig. 7 is an image indicating the identification information of the identified analyst.

Thereby, the analyst can visually check the identification information output to the terminal display unit 130. As a result, the analyst can confirm, for example, whether the voice device 10 worn by the analyst is connected to the terminal device 100a or the terminal device 100b.

### [Embodiment 2]

### [Configuration of Analysis Support System]

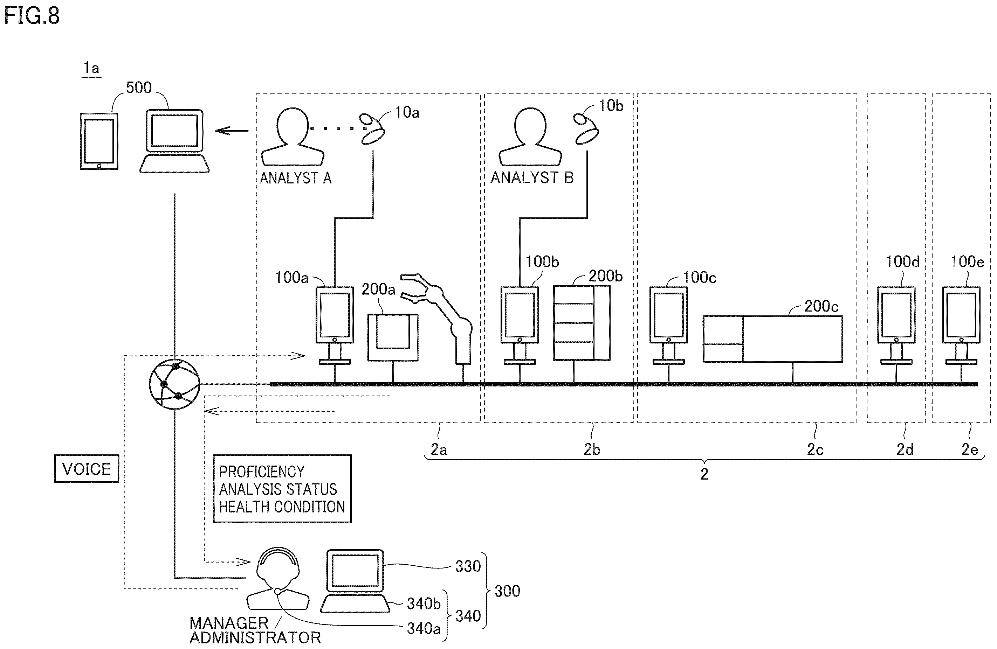

Fig. 8 is a diagram schematically showing a configuration of an analysis support system 1a according to Embodiment 2. The analysis support system 1a according to Embodiment 2 includes voice devices 10, terminal devices 100, and further includes a management device 300. Further, the analysis support system 1a includes a voice device 10a and a voice device 10b as the voice devices 10. The voice device 10a is worn by an analyst A, and the voice device 10b is worn by an analyst B. Further, the analysis support system 1a further includes terminal devices 100c to 100e as the terminal devices 100. The terminal device 100c is provided corresponding to an analysis apparatus 200c. An analysis apparatus to which the terminal device 100d corresponds and an analysis apparatus to which the terminal device 100e corresponds are not shown.

In the analysis support system 1a according to the present embodiment, the terminal devices 100a to 100e, the analysis apparatuses 200a to 200c, and the management device 300 are all connected to a communication network. The communication network may be either a wired communication network or a wireless communication network.

In Fig. 8, a predetermined range 2a of the analysis apparatus 200a to which the terminal device 100a corresponds is indicated by a dotted line. Similarly, for the terminal devices 100b to 100e, predetermined ranges 2b to 2e are indicated by dotted lines. At this time, analyst A is performing an analysis task on the analysis apparatus 200a, and analyst B is performing an analysis task on the analysis apparatus 200b. Since analyst A is within the predetermined range 2a of the analysis apparatus 200a, the terminal device 100a connects via wireless communication to the voice device 10a worn by analyst A. Similarly, since analyst B is within the predetermined range 2b of the analysis apparatus 200b, the terminal device 100b connects via wireless communication to the voice device 10b worn by analyst B.

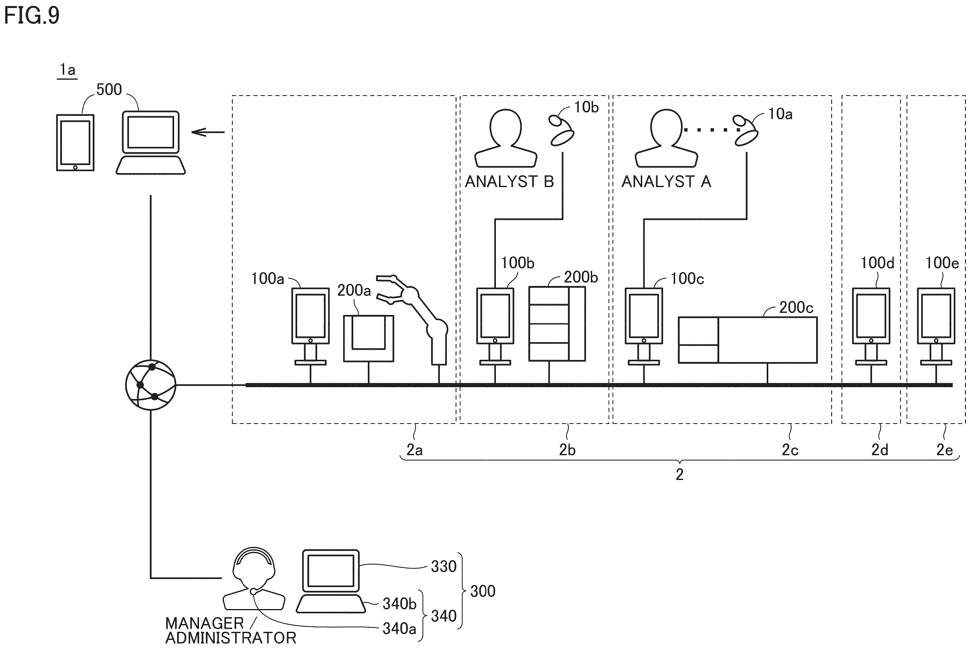

Fig. 9 is a diagram showing a state where analyst A has moved in the analysis support system 1a according to Embodiment 2. In Fig. 9, analyst A has moved to the predetermined range 2c of the analysis apparatus 200c. At this time, the terminal device 100a does not connect to the voice device 10a of analyst A, and the terminal device 100c connects to the voice device 10a of analyst A.

In the analysis support system 1a, since both the voice device 10a and the voice device 10b are open-type earphones, analyst A and analyst B can perform analysis tasks while communicating with each other by conversation.

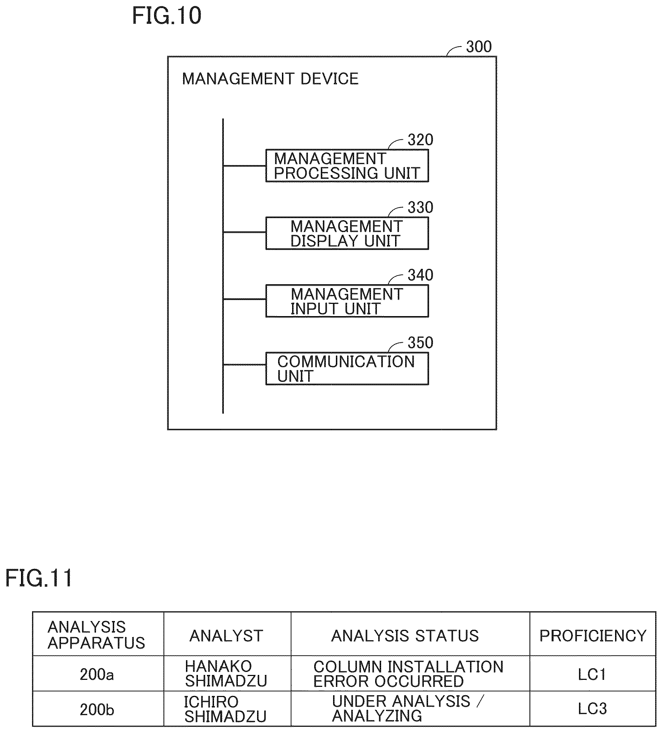

The management device 300 is used by a manager of the analysis tasks. Fig. 10 is a block diagram of the management device 300. The management device 300 includes a management processing unit 320, a management display unit 330, a management input unit 340, and a communication unit 350. The management display unit 330 displays an image. The management display unit 330 is, for example, a monitor. The management input unit 340 has a management input unit 340a and a management input unit 340b. The management input unit 340a is, for example, a headset. The management input unit 340b is, for example, a keyboard. Thereby, the management input unit 340 accepts input by voice and input by characters. The communication unit 350 connects the management device 300 to the terminal devices 100 and the analysis apparatuses 200 via the communication network.

The management processing unit 320 operates the management display unit 330, the management input unit 340, and the communication unit 350 according to a program. The management processing unit 320 is composed of, for example, a CPU (Central 'Processing Unit), an FPGA (Field Programmable Gate Array), a GPU (Graphics Processing Unit), or an MPU (Multi Processing Unit). Note that the management processing unit 320 may be composed of processing circuitry.

Each of the terminal devices 100c to 100e includes the same configuration as that of the terminal device 100a described in Embodiment 1. Note that the terminal devices 100a to 100e and the analysis apparatuses 200a to 200c in the analysis support system 1a all further include a communication unit for connecting to the communication network.

### [Analysis Support by Manager]

Processing related to analysis support using the management device 300 executed by the analysis support system 1a according to the present embodiment will be described.

First, the terminal device 100a executes the processing of steps S10 to S40 of Fig. 5 described in Embodiment 1, and causes the support information to be output to the voice device 10.

Next, the management processing unit 320 acquires the proficiency of analyst A in the analysis task of the analysis apparatus 200a from the terminal device 100a via the communication network.

Next, the management processing unit 320 acquires the analysis status of the analysis apparatus 200a from the analysis apparatus 200a via the communication network. The analysis status of the analysis apparatus 200a is, for example, under analysis, during column replacement, column installation error occurrence, or the like.

Then, the management processing unit 320 causes the proficiency of analyst A and the analysis status of the analysis apparatus 200a to be displayed on the management display unit 330.

Note that although the processing for analyst A has been described here, the proficiency of analyst B and the analysis status of the analysis apparatus 200b are similarly displayed on the management display unit 330 by similar processing for analyst B.

Fig. 11 is a diagram showing an example of an image displaying the proficiency of the analyst and the analysis status of the analysis apparatus. In Fig. 11, the analysis apparatus, the name which is the identification information of the analyst, the analysis status, and the proficiency of the analyst are associated with each other. Thereby, the manager can grasp the proficiency of the analyst and the analysis status of the analysis apparatus at a location distant from the location where the analysis apparatus 200 is located.

Thereafter, when the manager inputs voice via the management input unit 340a as advice for the analysis task of analyst A based on the display of the management display unit 330, the management processing unit 320 causes the voice to be output to the voice device 10a via the communication network. Further, when the manager inputs characters via the management input unit 340b as advice for the analysis task of analyst A, the management processing unit 320 causes the characters to be output to the terminal display unit 130 of the terminal device 100a via the communication network.

Thereby, the analyst can obtain advice from the manager based on the proficiency in the analysis task and the analysis status. For example, since the analyst "Hanako Shimadzu" has an analysis status of "Column installation error occurrence" and a low proficiency of "LC1", she can obtain advice on detailed procedures for resolving the error.

Furthermore, when the input unit 16 of the voice device 10 is a microphone, the analyst can talk with the manager using the input unit 16. Thereby, the analyst can proceed with the analysis task while confirming unclear procedures with the manager.

### <Modification 1 of Embodiment 2>

The analysis support system 1a according to Embodiment 2 may further allow the manager to grasp the health condition of the analyst. The processing in the analysis support system 1a in this case will be described below.

First, the terminal device 100a executes the processing of steps S10 to S35 of Fig. 5 described in Embodiment 1, and determines support information for the analysis task of the identified analyst.

Next, the terminal device 100a accepts an input of the physical condition of analyst A. Specifically, the terminal display unit 130 outputs an image for accepting an input of the physical condition of analyst A, and the terminal input unit 140 accepts the input of the physical condition of analyst A.

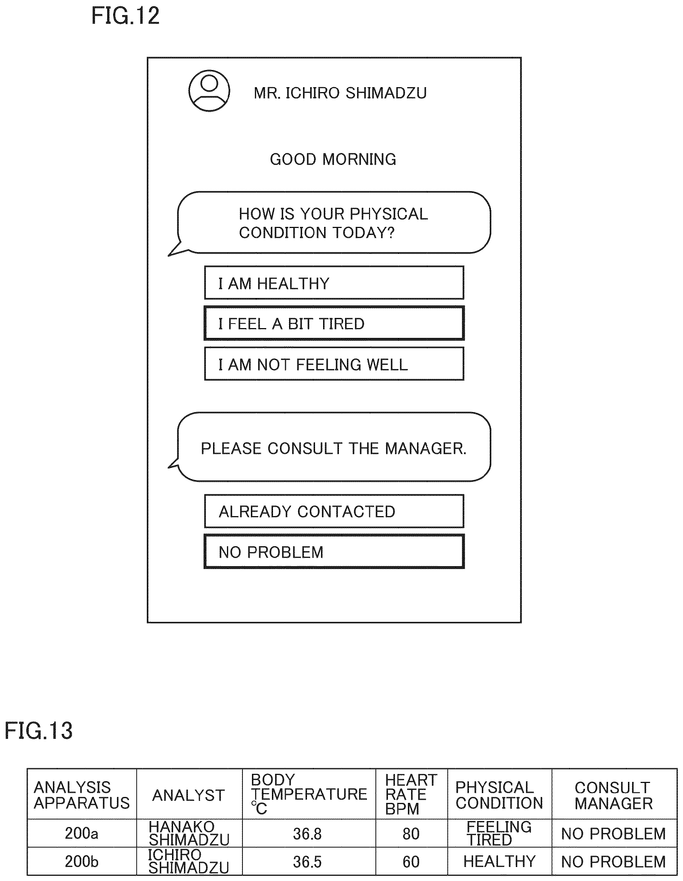

Fig. 12 is a diagram showing an example of an image for accepting an input of the physical condition of the analyst. The analyst selects one of "I am healthy", "I feel tired", and "I am not feeling well" shown in Fig. 12. Also, regarding the physical condition, the analyst selects "Already contacted" or "No problem" to the manager.

Next, the terminal device 100a causes the voice device 10 to output support information (Step S40).

Next, the terminal device 100a acquires measured values of the biological state of analyst A. Specifically, the terminal device 100a causes the voice device 10a to acquire measured values such as body temperature or heart rate, which are biological states of analyst A. Then, the terminal device 100a acquires the measured values of the biological state of analyst A from the voice device 10a via wireless communication.

Next, the management processing unit 320 acquires the measured values of the biological state of analyst A and the physical condition of analyst A from the terminal device 100a via the communication network.

Then, the management processing unit 320 causes the measured values of the biological state of analyst A and the physical condition of analyst A to be displayed on the management display unit 330.

Note that although the processing for analyst A has been described here, the management processing unit 320 causes the measured values of the biological state of analyst B and the physical condition of analyst B to be displayed on the management display unit 330 by similar processing for analyst B.

Fig. 13 is a diagram showing an example of an image indicating measured values of the biological state and the physical condition of the analyst. In Fig. 13, the analysis apparatus, the name which is the identification information of the analyst, the measured values of the biological state of the analyst, and the physical condition of the analyst are associated with each other. Thereby, the manager can grasp the health condition of the analyst at a location distant from the location where the analysis apparatus 200 is located. For example, since the analyst "Hanako Shimadzu" answers "No problem" although her physical condition is "Feeling tired", the manager can make a judgment such as to watch the situation for a while.

Thereafter, when the manager inputs voice via the management input unit 340a as advice for the analysis task of analyst A based on the display of the management display unit 330, the management processing unit 320 causes the voice to be output to the voice device 10a via the communication network. Further, when the manager inputs characters via the management input unit 340b as advice for the analysis task of analyst A, the management processing unit 320 causes the characters to be output to the terminal display unit 130 of the terminal device 100a via the communication network.

Thereby, the analyst can obtain instructions based on the health condition from the manager. For example, since the physical condition of the analyst "Hanako Shimadzu" is "Feeling tired", she can obtain instructions such as to take a break from the manager.

Furthermore, when the input unit 16 of the voice device 10 is a microphone, the analyst can talk with the manager using the input unit 16. Thereby, the manager can grasp the health condition of the analyst in more detail, for example, by asking questions to the analyst.

### <Modification 2 of Embodiment 2>

An analysis support system 1b according to Modification 2 of Embodiment 2 may further provide analysis support based on an analysis plan 400. The analysis plan 400 is a plan in which an analyst and an analysis task to be performed by the analyst are associated with each other for each analysis apparatus.

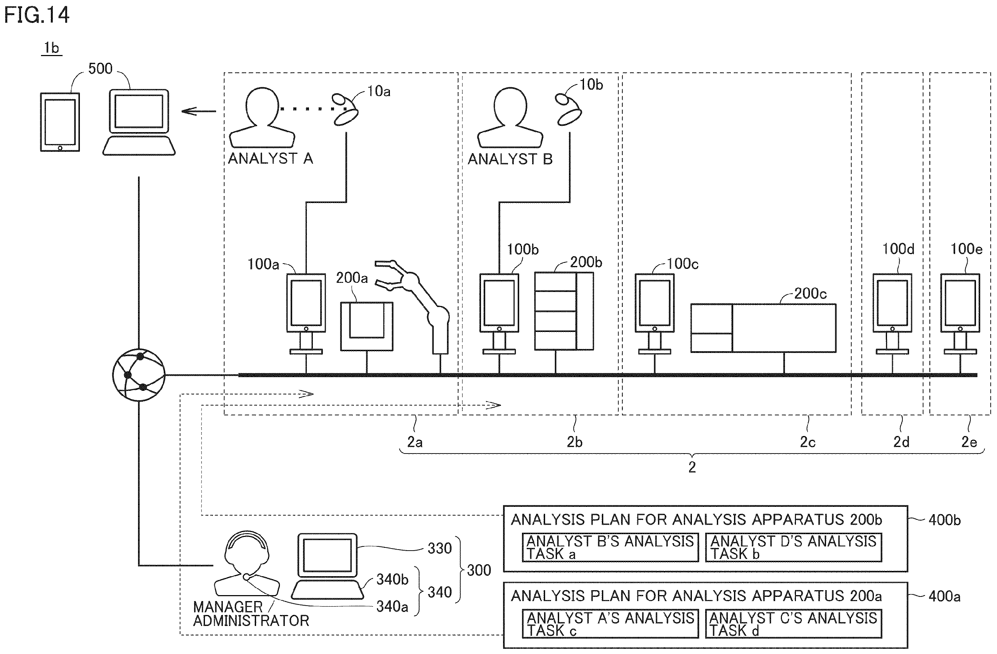

Fig. 14 is a diagram showing an example of processing of the analysis support system 1b according to Modification 2 of Embodiment 2. The analysis plan 400 in this modification is an analysis plan 400a and an analysis plan 400b. The analysis plan 400a is an analysis plan for the analysis apparatus 200a. The analysis plan 400a is a plan for analyst A to perform analysis task c, and for analyst C to perform analysis task d. The analysis plan 400b is an analysis plan for the analysis apparatus 200b. The analysis plan 400b is a plan for analyst B to perform analysis task a, and for analyst D to perform analysis task b.

The support information 111 in the analysis support system 1b includes a difficulty level for each analysis task. Specifically, the support information 111 of the terminal device 100a includes the difficulty level of the analysis task c and the difficulty level of the analysis task d of the analysis apparatus 200a. Also, the support information 111 of the terminal device 100b includes the difficulty level of the analysis task a and the difficulty level of the analysis task b of the analysis apparatus 200b. The difficulty level of the analysis task is, for example, a higher difficulty level if it is an analysis task that handles a sample requiring work with higher precision.

Further, the support information 111 in the analysis support system 1b includes support information that differs depending on the proficiency of the analyst and the difficulty level of the analysis task. For example, for a certain proficiency of an analyst, support information for an analysis task with a higher difficulty level includes more precautions for the analysis task.

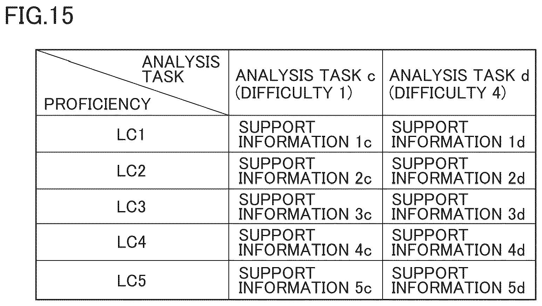

Fig. 15 is a diagram showing an example of the support information 111 possessed by the terminal device 100a according to Modification 2 of Embodiment 2. The support information differs depending on the proficiency of the analyst and the difficulty level of the analysis task of the analysis apparatus 200a. For example, since support information 1d is support information corresponding to a higher difficulty level than support information 1c, it includes more precautions. Also, since support information 2c is support information corresponding to a higher proficiency than support information 1c, it includes fewer precautions.

Thus, the support information according to the proficiency of the analyst and the difficulty level of the analysis task in the analysis support system 1b is support information necessary and sufficient for an analyst having that proficiency to accurately perform the analysis task of that difficulty level.

Processing related to analysis support based on the analysis plan 400 executed by the analysis support system 1b according to this modification will be described. Here, the analysis plan 400a among the analysis plan 400a and the analysis plan 400b will be described.

First, the management input unit 340b accepts an input of the analysis plan 400a. Specifically, the manager operates the management input unit 340b to input the analysis plan 400a.

Next, the management processing unit 320 transmits the accepted analysis plan via the communication network to the terminal device corresponding to the analysis apparatus for which the analysis task is planned. Specifically, the management processing unit 320 transmits the analysis plan 400a to the terminal device 100a.

On the other hand, the terminal device 100a first executes the processing of steps S10 to S30 of Fig. 5 described in Embodiment 1.

Next, the terminal device 100a identifies the difficulty level of the analysis task to be performed by analyst A. Specifically, the terminal device 100a first identifies that the analysis task to be performed by analyst A is analysis task c based on the analysis plan 400a. Then, the terminal device 100a identifies the difficulty level of analysis task c based on the support information 111.

Then, the terminal device 100a selects support information. Specifically, the terminal device 100a selects support information corresponding to the proficiency of analyst A in the analysis task of the analysis apparatus 200a and the difficulty level of analysis task c, based on the support information 111.

Finally, the terminal device 100a causes the selected support information to be output to the voice device 10a. Note that although the terminal device 100a has been described here, the terminal device 100b also executes similar processing to output support information based on the analysis plan 400b to the voice device 10b.

As described above, in the analysis support system 1b according to the present embodiment, support information for the analysis apparatus corresponding to the proficiency of the identified analyst and the difficulty level of the analysis task is selected. Thereby, the analyst can obtain support information corresponding to the difficulty level of the analysis task in addition to the proficiency of the analyst.

[Other Modifications] In Embodiments 1 and 2 described above, the terminal device 100 is provided separately from the analysis apparatus 200. However, the terminal device 100 may be included in the analysis apparatus 200. For example, a communication unit of the analysis apparatus 200 may also serve as the communication unit 150 of the terminal device 100, and the communication unit of the analysis apparatus 200 and the communication unit 22 of the voice device 10 may be connected via wireless communication.

In Embodiments 1 and 2 described above, the voice device 10 is an open-type earphone. However, the voice device 10 may be a closed-type earphone. Even in this case, the analyst can obtain support information while working. Further, the voice device 10 may be a bone conduction earphone. Even in this case, the analyst can perform the analysis task while listening to ambient sounds.

In Embodiments 1 and 2 described above, the proficiency of the analyst in the analysis task is a proficiency based on the past number of executions of the analysis task, the period from the previous analysis task, and the work achievement level of the previous analysis task. However, the proficiency may be based on any one or any two of these. Also in this case, since the proficiency is based on the analyst's past analysis tasks, the analyst can obtain support information necessary and sufficient for the analyst to work accurately.

In Embodiments 1 and 2 described above, the terminal processing unit 120 updates the proficiency of the analyst in the analysis task. However, the manager or the analyst may update the proficiency.

In Embodiments 1 and 2 described above, the proficiency of the analyst in the analysis task is updated every time the analysis task is performed. However, at this time, the updated proficiency may be the same as the proficiency before the update. For example, in step S60 of Fig. 5, if the work achievement level of the analysis task is high (e.g., level 4) but the number of executions is small (e.g., once), the terminal processing unit 120 may keep the updated proficiency the same as the proficiency before the update.

Both the analysis support systems of Embodiments 1 and 2 described above include a plurality of analysis apparatuses 200 and a plurality of terminal devices 100. However, the analysis support system may include one analysis apparatus 200 and one terminal device 100.

In Embodiments 1 and 2 described above, the terminal input unit 140 accepts the input of the analyst's work achievement level and the input of the analyst's physical condition. However, a personal terminal device 500 owned by the analyst may accept the input of the analyst's work achievement level and the input of the analyst's physical condition. The personal terminal device 500 is, for example, a PC or a smartphone.

In Embodiments 1 and 2 described above, the communication unit 150 of the terminal device 100 and the communication unit 22 of the voice device 10 are connected by Bluetooth. However, the present invention is not limited to this, and they may be connected by, for example, Wi-Fi. Also in this case, the communication unit 150 is set to connect with the voice device 10 on a one-to-one basis.

[Aspects] It will be understood by those skilled in the art that the above-described embodiments and modifications thereof are specific examples of the following aspects.

(Item 1) An analysis support system according to one aspect is an analysis support system for supporting an analysis task in an analysis apparatus, comprising: a voice device configured to be worn by each analyst using the analysis apparatus and to perform analysis support by voice; and a terminal device provided corresponding to the analysis apparatus and connected to the voice device via wireless communication. The terminal device includes: a storage unit configured to store support information for the analysis apparatus and analyst information including a proficiency of the analyst in the analysis task; a communication unit configured to authenticate wireless communication with the voice device of the analyst who is within a predetermined range of the analysis apparatus; and a terminal processing unit configured to identify the analyst authenticated by the communication unit and capable of wireless communication, and to output the support information for the analysis apparatus corresponding to the proficiency of the identified analyst to the voice device.

According to the analysis support system described in Item 1, the terminal processing unit outputs support information for the analysis apparatus corresponding to the proficiency of the identified analyst to the voice device worn by the analyst. Thereby, the analyst can obtain support information necessary and sufficient for the analyst to work accurately while working. As a result, the analysis support system can provide necessary analysis support to the analyst.

(Item 2) In the analysis support system described in Item 1, the terminal processing unit further stores the number of executions and the execution timing of the analysis task of the analyst in the storage unit, and updates the proficiency of the analyst information based on the past number of executions of the analysis task of the analyst and the period from the previous analysis task.

According to the analysis support system described in Item 2, the proficiency of the analyst can be set as a proficiency based on the past number of executions of the analysis task of the analyst and the period from the previous analysis task. Thereby, the analyst can obtain support information corresponding to the proficiency based on the analyst's past number of executions of the analysis task and the period from the previous analysis task.

(Item 3) In the analysis support system described in Item 1 or 2, the voice device is an open-type earphone.

According to the analysis support system described in Item 3, since the voice device is an open-type earphone, the analyst can perform the analysis task while listening to ambient sounds.

(Item 4) In the analysis support system described in any one of Items 1 to 3, the terminal device further includes a terminal display unit configured to display an image and a terminal input unit configured to accept an input from the analyst. The terminal device outputs an image of the support information to the terminal display unit in accordance with the support information for the analysis apparatus output to the voice device, and switches the support information for the analysis apparatus output to the voice device and the terminal display unit based on the input of the analyst accepted by the terminal input unit.

According to the analysis support system described in Item 4, since the image of the support information is output to the terminal display unit in accordance with the support information for the analysis apparatus output to the voice device, the analyst can also check the support information by the image. Further, since the support information for the analysis apparatus output to the voice device and the terminal display unit is switched based on the input of the analyst accepted by the terminal input unit, the analyst can switch the output support information in accordance with the progress of the actual analysis task.

(Item 5) In the analysis support system described in any one of Items 1 to 4, the terminal processing unit further outputs identification information of the identified analyst to the terminal display unit.

According to the analysis support system described in Item 5, the analyst can visually check the identification information output to the terminal display unit, and thereby can confirm that the voice device worn by the analyst is connected to the terminal device.

(Item 6) In the analysis support system described in any one of Items 1 to 5, the terminal input unit further accepts an input of a work achievement level of the analyst for the analysis task. The terminal processing unit updates the proficiency of the analyst in consideration of the work achievement level of the analyst accepted by the terminal input unit.

According to the analysis support system described in Item 6, the proficiency of the analyst can be set as a proficiency corresponding to the work achievement level of the analyst for the analysis task. Thereby, the analyst can obtain support information corresponding to the work achievement level of the analyst for the analysis task.

(Item 7) The analysis support system described in any one of Items 1 to 6 further comprises a management device connected to the terminal device and the analysis apparatus via a communication network. The management device includes: a management display unit configured to display an image; and a management processing unit configured to output the proficiency of the analyst in the analysis task acquired from the terminal device and an analysis status acquired from the analysis apparatus to the management display unit.

According to the analysis support system described in Item 7, the management display unit can display the proficiency of the analyst in the analysis task and the analysis status acquired from the analysis apparatus. Thereby, a user of the management device can grasp the proficiency of the analyst in the analysis task and the analysis status acquired from the analysis apparatus at a location distant from the location where the analysis apparatus is located.

(Item 8) In the analysis support system described in any one of Items 1 to 7, the terminal input unit further accepts an input of a physical condition of the analyst. The voice device further includes a measurement unit configured to acquire measured values of a biological state of the analyst. The terminal processing unit further acquires the measured values of the biological state of the analyst acquired by the measurement unit from the voice device. The management processing unit further outputs the physical condition and the measured values of the biological state of the analyst to the management display unit.

According to the analysis support system described in Item 8, the management display unit can display the physical condition and the measured values of the biological state of the analyst. Thereby, the user of the management device can grasp the health condition of the analyst performing the analysis task.

(Item 9) The analysis support system described in any one of Items 1 to 8 further comprises the analysis apparatus. The analysis apparatus includes the terminal device.

According to the analysis support system described in Item 9, the analysis support system can be configured to include the analysis apparatus including the terminal device.

(Item 10) The analysis support system described in any one of Items 1 to 9 further comprises a management device connected to the terminal device via a communication network. The management device includes: a management input unit configured to accept an input of an analysis plan in which an analyst and an analysis task to be performed by the analyst are associated with each other for each analysis apparatus; and a management processing unit configured to transmit the analysis plan accepted by the management input unit to the terminal device corresponding to the analysis apparatus. The terminal device further identifies the analysis task to be performed by the identified analyst based on the analysis plan transmitted from the management processing unit, and selects support information for the analysis apparatus corresponding to the proficiency of the identified analyst and a difficulty level of the identified analysis task.

According to the analysis support system described in Item 10, support information for the analysis apparatus corresponding to the proficiency of the identified analyst and the difficulty level of the identified analysis task is selected. Thereby, the analyst can obtain support information corresponding to the difficulty level of the analysis task in addition to the proficiency of the analyst.

(Item 11) An analysis support method according to one aspect is an analysis support method for supporting an analysis task in an analysis apparatus in an analysis support system comprising a voice device configured to be worn by each analyst using the analysis apparatus and to perform analysis support by voice, and a terminal device provided corresponding to the analysis apparatus and connected to the voice device via wireless communication, the method including: a step of authenticating wireless communication with the voice device of an analyst who is within a predetermined range of the analysis apparatus; a step of identifying the authenticated analyst capable of wireless communication from analyst information stored in a storage unit of the terminal device; and a step of determining a proficiency of the analyst identified from the analyst information stored in the storage unit, reading support information for the analysis apparatus corresponding to the proficiency from the storage unit, and outputting the support information to the voice device.

According to the analysis support method described in Item 11, support information for the analysis apparatus corresponding to the proficiency of the identified analyst is output to the voice device worn by the analyst. Thereby, the analyst can obtain support information necessary and sufficient for the analyst to work accurately while working. As a result, the analysis support method can provide necessary analysis support to the analyst.

(Item 12) A program according to one aspect causes a computer to execute the analysis support method described in Item 11.

According to the program described in Item 12, support information for the analysis apparatus corresponding to the proficiency of the identified analyst is output to the voice device worn by the analyst. Thereby, the analyst can obtain support information necessary and sufficient for the analyst to work accurately while working. As a result, the program can provide necessary analysis support to the analyst.

The embodiments disclosed this time should be considered as illustrative in all points and not restrictive. The scope of the present invention is shown not by the above description of the embodiments but by the claims, and it is intended that all modifications within the meaning and scope equivalent to the claims are included.

### [Reference Signs List]

1, 1a, 1b: Analysis support system 111: Support information 2a, 2b, 2c, 2d, 2e: Predetermined range 10, 10a, 10b: Voice device 12: Sound output unit 14: Light output unit 16: Input unit 18: Control unit 20: Measurement unit 22, 150, 350: Communication unit 100, 100a, 100b, 100c, 100d, 100e: Terminal device 110: Storage unit 112: Analyst information 113: Analysis support program 120: Terminal processing unit 130: Terminal display unit 140: Terminal input unit 200, 200a, 200b, 200c: Analysis apparatus 300: Management device 320: Management processing unit 330: Management display unit 340, 340a, 340b: Management input unit 400, 400a, 400b: Analysis plan 500: Personal terminal device

## Claims

1. An analysis support system (1) for supporting an analysis task in an analysis apparatus (200), comprising: a voice device (10) configured to be worn by each analyst using the analysis apparatus (200) and to perform analysis support by voice; and a terminal device (100) provided corresponding to the analysis apparatus (200) and connected to the voice (10) device via wireless communication, wherein the terminal device (100) includes: a storage unit (110) configured to store support information (111) for the analysis apparatus (200) and analyst information (112) including a proficiency of the analyst in the analysis task; a communication unit (22) configured to authenticate wireless communication with the voice device (10) of the analyst who is within a predetermined range (2a) of the analysis apparatus (200); and a terminal processing unit (120) configured to identify the analyst authenticated by the communication unit (22) and capable of wireless communication (S15), and to output the support information (111) for the analysis apparatus (200) corresponding to the proficiency of the identified analyst to the voice device (10) (S40).

2. The analysis support system according to claim 1, wherein the terminal processing unit further stores the number of executions and the execution timing of the analysis task of the analyst in the storage unit, and updates the proficiency of the analyst information based on the past number of executions of the analysis task of the analyst and a period from a previous analysis task.

3. The analysis support system according to claim 1 or 2, wherein the voice device is an open-type earphone.

4. The analysis support system according to any one of the preceding claims, wherein the terminal device further includes a terminal display unit (130) configured to display an image and a terminal input unit (140) configured to accept an input from the analyst, outputs an image of the support information to the terminal display unit in accordance with the support information for the analysis apparatus output to the voice device, and switches the support information for the analysis apparatus output to the voice device and the terminal display unit based on the input of the analyst accepted by the terminal input unit.

5. The analysis support system according to claim 4, wherein the terminal processing unit further outputs identification information of the identified analyst to the terminal display unit.

6. The analysis support system according to claim 4 or 5, wherein the terminal input unit further accepts an input of a work achievement level of the analyst for the analysis task (S50), and the terminal processing unit updates the proficiency of the analyst in consideration of the work achievement level of the analyst accepted by the terminal input unit (S60).

7. The analysis support system according to any one of the preceding claims, further comprising a management device (300) connected to the terminal device and the analysis apparatus via a communication network, wherein the management device includes: a management display unit (330) configured to display an image; and a management processing unit (320) configured to output the proficiency of the analyst in the analysis task acquired from the terminal device and an analysis status acquired from the analysis apparatus to the management display unit.

8. The analysis support system according to claim 7, wherein the voice device further includes a measurement unit (20) configured to acquire measured values of a biological state of the analyst, the terminal device further includes a terminal input unit (140) configured to accept an input of a physical condition of the analyst, the terminal processing unit further acquires the measured values of the biological state of the analyst acquired by the measurement unit from the voice device, and the management processing unit further outputs the physical condition and the measured values of the biological state of the analyst to the management display unit.

9. The analysis support system according to any one of the preceding claims, further comprising the analysis apparatus, wherein the analysis apparatus includes the terminal device.

10. The analysis support system according to any one of the preceding claims, further comprising a management device (300) connected to the terminal device via a communication network, wherein the management device includes: a management input unit (340) configured to accept an input of an analysis plan (400) in which an analyst and an analysis task to be performed by the analyst are associated with each other for each analysis apparatus; and a management processing unit (320) configured to transmit the analysis plan accepted by the management input unit to the terminal device corresponding to the analysis apparatus, and wherein the terminal device further identifies the analysis task to be performed by the identified analyst based on the analysis plan transmitted from the management processing unit, and selects support information for the analysis apparatus corresponding to the proficiency of the identified analyst and a difficulty level of the identified analysis task.

11. An analysis support method for supporting an analysis task in an analysis apparatus in an analysis support system comprising a voice device configured to be worn by each analyst using the analysis apparatus and to perform analysis support by voice, and a terminal device provided corresponding to the analysis apparatus and connected to the voice device via wireless communication, the method comprising: a step of authenticating wireless communication with the voice device of an analyst who is within a predetermined range of the analysis apparatus (S 1 0); a step of identifying the authenticated analyst capable of wireless communication from analyst information stored in a storage unit of the terminal device (S15); and a step of determining a proficiency of the analyst identified from the analyst information stored in the storage unit, reading support information for the analysis apparatus corresponding to the proficiency from the storage unit, and outputting the support information to the voice device (S40).

12. A program for causing a computer to execute the analysis support method according to claim 11.
